# EUROPEAN PATENT APPLICATION

(11) **EP 1 938 800 A1**
(43) Date of publication of application: **02.07.2008**
(21) Application number: 07122542.9
(22) Date of filing: 06.12.2007
(51) Int. Cl.: A61K 9/10, A61K 9/14, A61K 31/436

(54) **Sirolimus nanodispersion**

(30) Priority: 06.12.2006 IN DE26142006
(71) Applicant: Ranbaxy Laboratories Limited, Haryana 122001, Delhi (IN)
(72) Inventor: Raheja, Praveen, New Delhi - 110058 (IN); Kaushik, Atul, Uttar Pradesh 201001 (IN); Gandhi, Rajesh, Haryana - 122001 (IN); Singh, Romi Barat, Uttar Pradesh - 221002 (IN); Mathur, Rajeev Shanker, Haryana - 122002 (IN)
(74) Representative: Cronin, Brian Harold John

(57) **Abstract**

The present invention relates to a nanodispersion composition comprising sirolimus and a surface modifier wherein effective average particle size of Sirolimus is more than 400 nm and process for preparation thereof.

## Description

### Field of the Invention

The present invention relates to a nanodispersion composition comprising sirolimus and a surface modifier wherein effective average particle size of Sirolimus is more than 400 nm and process for preparation thereof. It, further, relates to a pharmaceutical composition comprising the said nanodispersion.

### Background of the Invention

Rapamycin is a macrolide antibiotic produced by Streptomyces hygroscopicus which was discovered first for its properties as an antifungal agent. It adversely affects the growth of fungi such as Candida albicans and Microsporum gypseum. Rapamycin, its preparation and its antibiotic activity were described in U.S. 3,929,992. In 1977 Martel, et al. reported immunosuppressive properties of rapamycin against experimental allergic encephalitis and adjuvant arthritis in the Canadian Journal of Physiological Pharmacology, 55, 48-51 (1977). In 1989, Calne, R. Y. et al. in Lancet, 1989, no. 2, p. 227 and Morris, R. E. and Meiser, B. M. in Medicinal Science Research, 1989, No. 17, P. 609-10, separately reported on the effectiveness of rapamycin in inhibiting rejection *in vivo* in allograft transplantation. U.S. 5,100,899 contains description of the use of Rapamycin to inhibit transplantation rejection in mammals.

Its poor water solubility poses an issue in formulating the drug into suitable dosage form. In addition, it has been reported that compositions of Sirolimus with conventional excipients can show unpredictable dissolution rates, irregular bioavailability profiles, as well instability problems. Currently, Sirolimus is available in two dosage forms, namely tablet and oral solution.

U.S, 5,516,770 and 5,530,006 relate to intravenous rapamycin formulations, and U.S. 5,536,729 and 5,559,121 relates to liquid oral rapamycin formulations.

U.S. 6,004,973 relates to a pharmaceutical composition containing a solid dispersion comprising rapamycin and carrier medium in the form of co-precipitates.

U.S. 5,989,591 and U.S. 5,985,325 relates to a solid dosage unit ofrapamycin claimed as a core, which is over coated with rapamycin, and a sugar coat containing polaxamer 188; optionally povidone and ,microcrystalline cellulose; and sucrose.

U.S. 5,145,684 relates to a nanoparticulate composition comprising particles consisting of a poorly soluble drug having adsorbed onto the surface thereof a non-crosslinked surface stabilizer wherein effective average particle size of drug substance is less than about 400 nm.

### Summary of the Invention

We have now developed a nanodispersion comprising Sirolimus and surface modifiers wherein the effective average particle size of Sirolimus is above 400 nm.

Also, we have developed a solid pharmaceutical composition comprising sirolimus prepared by wet granulation method, which is a conventionally used method for the preparation of solid pharmaceutical compositions.

Hence, in one of the aspect, there is provided a nanodispersion comprising Sirolimus and a surface modifier wherein the effective average particle size of Sirolimus is more than 400nm.

In another aspect, there is provided a process for preparing nanodispersion comprising Sirolimus and a surface modifier, comprising the steps of:
a) Dissolving or dispersing a surface modifier in a solvent;
b) Dispersing Sirolimus in the dispersion/solution of step a); and
c) Wet milling the dispersion to obtain Sirolimus having an effective average particle size of more than 400 nm.

In another aspect, there is provided a pharmaceutical composition comprising a nanodispersion comprising Sirolimus and a surface modifier wherein the effective average particle size of Sirolimus is more than 400nm.

In another aspect, there is provided a process for preparing a pharmaceutical composition comprising a nanodispersion comprising Sirolimus and a surface modifier comprising the steps of:
a) Dissolving or dispersing a surface modifier in a solvent;
b) Dispersing Sirolimus in the dispersion/solution of step a);
c) Wet milling the dispersion to obtain Sirolimus having an effective average particle size of more than 400 nm; and
d) Processing the nanodispersion into a suitable pharmaceutical composition.

In another aspect, there is provided a pharmaceutical composition comprising a nanodispersion comprising Sirolimus and a surface modifier wherein the effective average particle size of Sirolimus is more than 400 nm and the said composition is prepared by a granulation method.

In another aspect, there is provided a method of preparing a pharmaceutical composition comprising a nanodispersion comprising Sirolimus and a surface modifier comprising the steps of:
a) Dissolving or dispersing a surface modifier in a solvent,
b) Dispersing Sirolimus in the dispersion/solution of step a) and
c) Wet milling the dispersion to obtain Sirolimus having an effective average particle size of more than 400 nm,
d) Blending other pharmaceutically acceptable excipients selected from group consisting of sugar, binders, diluents, lubricant/glidant, disintegrating agent, antioxidants and coloring agents,
e) Granulating the excipients of step d) with dispersion of step c),
f) Drying and sizing the wet granules,
g) Optionally, blending said granules with extra granular excipients,
h) Processing into suitable pharmaceutical composition.

In another aspect, there is provided a pharmaceutical composition comprising
a) a nanodispersion comprising Sirolimus and surface modifiers
b) an inert core coated with said nanodipersion
wherein effective average particle size of Sirolimus is more than 400 nm.

In another aspect, there is provided a method of preparing a pharmaceutical composition comprising a nanodispersion comprising Sirolimus and surface modifiers comprising the steps of:
a) Dissolving or dispersing a surface modifier in a solvent,
b) Dispersing Sirolimus in dispersion/solution of step a) and
c) Wet milling the dispersion to obtain effective average particle size of Sirolimus more than 400 nm,
d) Coating the inert core with Sirolimus dispersion of step c),
e) Optionally, further processing the drug coated cores into suitable composition.

In another aspect, there is provided a method of treatment of organ or tissue transplant rejection, autoimmune disease, inflammatory conditions, or multi-drug resistance, the method comprising: orally administering to a subject a pharmaceutical composition comprising a nanodispersion comprising Sirolimus and surface modifiers wherein effective average particle size of Sirolimus is more than 400 nm.

### Detailed Description

"Sirolimus" as employed herein is intended to include amorphous or crystalline form of the drug. The crystalline form may include polymorph form I or II or a mixture thereof. Sirolimus may be present in the composition in an amount of about 0.001 to about 20% by weight, based on the total weight of the composition.

The nanodispersion of Sirolimus comprises Sirolimus particles having an average particle size greater than 400 nm, more particularly 400 - 1200 nm, particularly 400-1000 nm. The nanodispersion may also comprise a part of drug in solubilized form. The term "effective average particle size" as used herein means 90% of the particles have a volume average particle size of more than 400 nm when measured by a suitable technique, also represented by d₉₀. Laser diffraction method which involves particle size measurement using light and generates distribution based on the volume may be used for the same, of which most commonly used is Malvern.

Wet milling is used to prepare nanodispersion of desired particle size. Methods of making finely divided drugs have been studied and efforts have been made in past to control the size and size range of drug particles in pharmaceutical composition. For example, dry milling techniques have been used to reduce the particle size. However, in conventional dry milling the limit of fineness is reached in the region of 1 microns whereas wet milling is beneficial in further reducing the particle size. Moreover, wet milling is also suitable for drugs such as immunosupressants wherein it is necessary to control the dust produced.

Various mills are available for wet milling the drug such as ball mill, an attritor mill, a vibratory mill, and media mills such as a sand mill and a bead mill. The grinding media used in wet milling includes zirconium oxide, such as 95% ZrO stabilized with magnesia, zirconium silicate, and glass grinding media, stainless steel, titania, alumina, and 95% ZrO stabilized with yttrium.

### "Pharmaceutical composition" as used herein includes tablet, capsule, granules and pills. "Inert core" as used herein includes placebo tablet core or inert beads or spheres.

The term "surface modifiers" as used herein means agents which are used to disperse the drug in a particular solvent and also enhance wetting properties of the drug. Such excipients include various polymers, low molecular weight oligomers, natural products and surfactants. Representative examples include gelatin, casein, lecithin (phosphatides), gum acacia, cholesterol, tragacanth, stearic acid, benzalkonium chloride, calcium stearate, colloidol silicon dioxide, phosphates, carboxymethylcellulose calcium, carboxymethylcellulose sodium, methylcellulose, hydroxyethylcellulose, hydroxypropylcellulose, hydroxypropylmethycellulose phthalate, noncrystalline cellulose, magnesium aluminum silicate, triethanolamine, polyvinyl alcohol, and polyvinylpyrrolidone. Surfactants include both non-ionic and ionic (cationic, anionic and zwitterionic) surfactants suitable for use in pharmaceutical dosage forms. These include polyethoxylated fatty acids and its derivatives, for example polyethylene glycol 400 distearate, polyethylene glycol-20 dioleate, polyethylene glycol 4-150 mono dilaurate, polyethylene glycol-20 glyceryl stearate; alcohol-oil transesterification products, for example polyethylene glycol-6 corn oil; polyglycerized fatty acids, for example polyglyceryl-6 pentaoleate; propylene glycol fatty acid esters, for example propylene glycol monocaprylate; mono and diglycerides for example glyceryl ricinoleate; fatty acids and their esters such as glyceryl monooleate, sterol and sterol derivatives; sorbitan fatty acid esters and its derivatives, for example polyethylene glycol-20 sorbitan monooleate, sorbitan monolaurate; polyethylene glycol alkyl ether or phenols, for example polyethylene glycol-20 cetyl ether, polyethylene glycol-10-100 nonyl phenol; sugar esters, for example sucrose monopalmitate; polyoxyethylene-polyoxypropylene block copolymers known as "poloxamer"; ionic surfactants, for example sodium caproate, sodium glycocholate, soy lecithin, sodium stearyl fumarate, propylene glycol alginate, octyl sulfosuccinate disodium, palmitoyl camitine. These may be used from the same category or a combination of surfactant with low molecular weight oligomers/natural products. The surface modifiers may be present in an amount from 0.1-10% by weight, based on the total weight of the composition.

The term "pharmaceutically acceptable excipients" as used herein include sugars, binders, diluents, lubricant/glidant, disintegrating agent, antioxidants and coloring agents. These excipients may be present as intragranularly or extragranularly.

Sugars may be used to prepare syrup dispersion, wherein the syrup dispersion comprises nanodispersion of the drug and sugars. Sugar may include lactose, mannitol, sorbitol, starch, sucrose and mixtures thereof.

Specific examples of "binders" include methyl cellulose, hydroxypropyl cellulose, hydroxyl propyl methycellulose, polyvinylpyrrolidone, gelatin, gum Arabic, ethyl cellulose, polyvinyl alcohol, pullulan, pregelatinized starch, agar, tragacanth, carboxymethyl cellulose, sodium alginate, propylene glycol, microcrystalline cellulose or mixtures thereof. The binders may be present in an amount from 0.1-20% by weight, based on the total weight of the composition.

The term "diluents" as used herein includes calcium carbonate, calcium phosphatedibasic, calcium phosphate-tribasic, calcium sulfate, cellulose-microcrystalline, cellulose powdered, dextrates, dextrins, dextrose excipients, fructose, kaolin, lactitol, lactose, mannitol, sorbitol, starch, sucrose and mixtures thereof.

Specific examples of lubricants/glidants include colloidal silicon dioxide, stearic acid, magnesium stearate, calcium stearate, talc, hydrogenated castor oil, and mixtures thereof.

Disintegrants preferred for the present invention may be selected from starches or modified starches such as starch, modified starch, croscarmellose sodium, crospovidone and sodium starch glycolate.

The composition may further comprise antioxidant, to protect the drug from oxidative degradation. Antioxidants may be selected from group consisting of ascorbic acid, sodium pyrosulphite, glutathion or sorbic acid.

Coloring agent may be selected from FDA approved colorants and the examples are Iron oxide, Lake of Tartrazine, Allura red, Lake of Quinoline yellow, Lake of Erythrosine.

The solvent used to prepare the dispersion may be selected from water, ethanol, isopropyl alcohol, ether or mixtures thereof
According to one of the embodiment, granules are prepared using high shear mixer granulator comprising the steps of:
a) Preparing nanodispersion of Sirolimus,
b) Blending the excipients
c) Granulating the excipients of step b) with nanodispersion of step a) in a high shear mixer granulator
d) Drying and sizing the granules,
e) Optionally, blending said granules with said extra granular excipients.
f) Compressing the blend into tablet or filling the blend into capsules.

According to another embodiment, granules are prepared using fluidized bed granulator comprising the steps of:
a) Preparing nanodispersion of Sirolimus,
b) Blending the excipients
c) Contacting Sirolimus nanodispersion with blend of step b) within a fluid bed granulator and selecting fluidising air flow and processing temperature and time to provide mixing and shearing action to form a granules,
d) Drying and sizing the granules,
e) Optionally, blending said granules with said extra granular excipients.
f) Compressing the blend into tablet or filling the blend into capsules.

According to another embodiment, the pharmaceutical composition of Sirolimus is prepared by a process comprising the step of:
a) blending pharmaceutically acceptable excipients;
b) optionally, granulating the blend of step a);
c) compressing the blend or the granules to obtain an inert tablet core;
d) dissolving or dispersing a surface modifier in a solvent;
e) dispersing Sirolimus in the dispersion/solution of step a);
f) wet milling the dispersion to obtain the desired particle size of Sirolimus;
g) coating the inert tablet core with Sirolimus dispersion of step f).
The invention is further illustrated by the following examples but they should not be construed as limiting the scope of this invention in any way.

### EXAMPLES

### EXAMPLE 1

| Ingredients | Qty/tab(mg) |
|---|---|
| **Nanodispersion** | |
| Sirolimus | 2 |
| Poloxamer-188 | 1 |
| Glyceryl monooleaate | 0.2 |
| Water | Q.S |

| **Syrup dispersion** | |
|---|---|
| Povidone | 2 |
| Sucrose | 120 |
| PEG-20000 | 5 |
| Water | QS |
| Microcrystalline Cellulose | 140 |

| **Extragranular** | |
|---|---|
| Microcrystalline Cellulose | 26.3 |
| Sodium Stearyl fumarate | 3.5 |
| Target weight | 300 |

### Procedure-

1. Poloxamer-188 was dissolved in water.
2. Glyceryl monooleate was dispersed in solution of step 1.
3. Sirolimus was dispersed in dispersion of step 2 under stirring.
4. The dispersion of step 3 was milled in dyno mill.
5. Povidone, sucrose and PEG was added into the dispersion of step 4.
6. Water is added to dispersion of step 5 to obtain consistency suitable for coating.
7. The dispersion of step 6 was loaded onto microcrystalline cellulose to obtain drug loaded granules.
8. Drug loaded granules of step 7 granules were dried and sifted.
9. Microcrystalline cellulose (extragranular) was added to drug loaded granules and lubricated using Sodium stearyl fumarate.
10. Lubricated blend is then compressed into suitable size tablet.

### EXAMPLE 2

| Ingredients | Qty/tab(mg) |
|---|---|
| **Nanodispersion** | |
| Sirolimus | 2 |
| Tween-80 | 1 |
| Glyceryl monooleaate | 0.2 |
| Water | Q.S |

| **Syrup dispersion** | |
|---|---|
| Povidone | 2 |
| Sucrose | 120 |
| PEG-20000 | 5 |
| Water | QS |
| Microcrystalline cellulose | 140 |

| **Extragranular** | |
|---|---|
| Microcrystalline cellulose | 26.3 |
| Sodium stearyl fumarate | 3.5 |
| Target weight | 300 |

Composition of Example 2 was prepared by the same process as described in Example 1.

### EXAMPLE 3

| | Ingredients | |
|---|---|---|
| **A** | **Inert core** | **mg/tablet** |
| | Lactose | 148.5 |
| | Magnesium stearate | 1.5 |
| | Tablet weight (mg) | 150 |

| **B)** | **Seal coating** | |
|---|---|---|
| | Pharmaceutical glaze (Shellac solution 50% w/v) | 11.25 |
| | Talc | q.s |
| | Absolute alcohol | q.s to make 25 % solution |

| **C** | **Sub Coat** | |
|---|---|---|
| | Sucrose | 31.45 |
| | Calcium Sulfate | 10.64 |
| | Microcrystalline cellulose | 3.87 |
| | PEG-20000 | 0.96 |
| | Titanium dioxide | 0.96 |
| | Talc | q.s |

| **D** | **Nanodispersion** | |
|---|---|---|
| | Sirolimus | 2 |
| | Tween-80 | 1 |
| | Water | q.s |

| **E)** | **Drug Coating** | |
|---|---|---|
| | Nanodispersion | 3 |
| | Microcrystalline cellulose | 1.48 |
| | Povidone | 0.74 |
| | Sucrose | 142.78 |
| | Water | q.s. |

### Procedure:

**A. Preparation of Inert core**
   i) Lactose and magnesium stearate were blended together and compressed into tablet.
**B. Seal Coating**
   i) Pharmaceutical glaze was diluted to 25%w/w solution by absolute alcohol.
   ii) Inert tablet cores of step A were coated with solution of Pharmaceutical glaze.
**C. Sub Coating**
   i) All the ingredients of the sub coat were dispersed in water to obtain a 70 %w/w of sub coat suspension.
   ii) The suspension of step i) was used to coat seal coated inert tablet cores.
**D. Preparation of Nanodispersion**
   i) Tween-80 was dissolved in water.
   ii) Sirolimus was dispersed in solution of step i) under stirring.
   iii) The dispersion of step ii) was milled in dyno mill.
**E. Preparation of Active coat**
   i) Nanodispersion was added into water.
   ii) Sucrose was added to dispersion of step i).
   iii) Povidone and microcrystalline cellulose were added to dispersion of step ii).
   iv) The resulting dispersion coated onto Sub coated tablets.

Particle size of the nanodispersion produced at step D was measured using Malvern particle size analyzer, d₉₀ was found to be 742 nm, d50 was found to be 182 nm.

### EXAMPLE 4

| | Ingredients | |
|---|---|---|
| **A )** | **Inert core** | **mg/tablet** |
| | Lactose | 129 |
| | PEG-6000 | 15 |
| | Talc | 3 |
| | Magnesium stearate | 3 |
| | Tablet weight (mg) | **150** |

| **B)** | **Seal coating** | |
|---|---|---|
| | Shellac | 4.5 |
| | Talc | q.s |
| | Absolute alcohol | q.s to make 25 % solution |
| **C )** | **Sub Coat** (Sucrose-65%, Calcium Sulfate-22%, MCC-8%, Macrogol/PEG-20000-2% and Titanium dioxide-2%) | 38.6 |

| **D )** | **Nanonized API** | |
|---|---|---|
| | API | 2 |
| | Poloxamer-407 | 1 |
| | Water | q.s |

| **E)** | **Sugar Barrier coat** | |
|---|---|---|
| | Sucrose | 10 |
| | PVP K-30 | 0.10 |
| **F)** | **Coating (Active layering)** | **2 mg strength** |
| | Nanonized API (dispersion in water) | 3 |
| | PVP K-30 (Povidone) | 0.63 |
| | Sucrose | 126.0 |
| | Water | q.s. |
| | Tocopherol | 0.5 |

| **G )** | **Sugar Barrier coat** | |
|---|---|---|
| | Sucrose | 10 |
| | PVP K-30 (Povidone) | 0.1 |
| **H )** | **Sub coat** (Sucrose-65%, Calcium Sulfate-22%, MCC-8%, Macrogol/PEG-20000-2% and Titanium dioxide-2%)* | 20 |

| **I)** | Polishing | |
|---|---|---|
| | Carnauba wax | 1.0 |
| | Chloroform | QS |

### Procedure:

**A. Preparation of Inert core**
   i) Lactose and PEG-6000 were sifted through sieve.
   ii) Talc and Magnesium stearate were sifted through sieve.
   iii) Blends of step i) and step ii) were blended together and compressed into suitable size tablets.
**B. Seal Coating**
   i) Pharmaceutical glaze was diluted to 25%w/w solution by absolute alcohol.
   ii) Inert tablet cores of step A were coated with solution of Pharmaceutical glaze.
**C. Sub Coating**
   i) All the ingredients of the sub coat were dispersed in water to obtain a 70 %w/w of sub coat suspension.
   ii) The suspension of step i) was used to coat seal coated inert tablet cores.
**D. Preparation of Nanodispersion**
   i) Poloxamer-407 was dissolved in water.
   ii) Sirolimus was dispersed in solution of step i) under stirring.
   iii) The dispersion of step ii) was milled in dyno mill.
**E. Preparation of Active coat**
   i) Nanodispersion was added into water.
   ii) Tocopherol was sprayed onto the sucrose.
   iii) Sucrose of step ii) was added to the dispersion of step i).
   iv) Povidone was added to dispersion of step iii).
   v) The resulting dispersion coated onto Sub coated tablets.
**F. Sugar Barrier Coat**
   i) Sugar and povidone were dispersed in water.
   ii) The dispersion of step i) was coated onto drug coated cores.
**G. Sub Coating**
   i) All the ingredients of the sub coat were dispersed in water to obtain a 70 %w/w of sub coat suspension.
   ii) The suspension of step i) was used to coat tablets of step F.
**I. Polishing**
   i) Solution of carnauba wax was prepared in Chloroform and was used for polishing sub coated tablets of step G.
      Particle size of the nanodispersion produced at step D was measured using Malvern particle size analyzer, d₉₀ was found to be 2.189 µm, d50 was found to be 0.293 µm.

## Claims

1. A nanodispersion of Sirolimus comprising Sirolimus and a surface modifier wherein the effective average particle size of Sirolimus is more than 400 nm.

2. The nanodispersion of Sirolimus according to claim 1 wherein the effective average particle size is 400-1200 nm.

3. The nanodispersion of Sirolimus according to claim 1 wherein the surface modifier is selected from the group consisting of polymers, low molecular weight oligomers, natural products and surfactants or mixture thereof.

4. The nanodispersion of Sirolimus according to claim 3 wherein the surfactant is selected from group consisting of polyethoxylated fatty acids and its derivatives, alcohol-oil transesterification products, propylene glycol fatty acid esters, sterol and sterol derivatives; sorbitan fatty acid esters and its derivatives, sugar esters, poloxamer, sodium caproate, sodium glycocholate, soy lecithin, sodium stearyl fumarate, propylene glycol alginate, octyl sulfosuccinate disodium, palmitoyl camitine or mixtures thereof.

5. The nanodispersion of Sirolimus according to claim 1 wherein the nanodispersion is prepared by a process comprising the steps of:
a) Dissolving or dispersing a surface modifier in a solvent;
b) Dispersing Sirolimus in the dispersion or solution of step a);
c) Wet milling the dispersion of step b) to obtain the desired particle size.

6. The nanodispersion of Sirolimus according to claim 5 wherein the solvent is selected from the group consisting of water, ethanol, isopropyl alcohol, ether or mixtures thereof.

7. The nanodispersion according to claim 5 wherein wet milling is carried out using a ball mill, an attritor mill, a vibratory mill or media mill.

8. A pharmaceutical composition of Sirolimus comprising nanodispersion of Sirolimus and a surface modifier wherein effective average particle size of Sirolimus is 400-1200 nm.

9. The pharmaceutical composition of Sirolimus according to claim 8 wherein the surface modifier is selected from the group consisting of polymers, low molecular weight oligomers, natural products and surfactants.

10. The pharmaceutical composition of Sirolimus according to claim 9 wherein the surfactant is selected from group consisting of polyethoxylated fatty acids and its derivatives, alcohol-oil transesterification products, propylene glycol fatty acid esters, sterol and sterol derivatives; sorbitan fatty acid esters and its derivatives, sugar esters, poloxamer, sodium caproate, sodium glycocholate, soy lecithin, sodium stearyl fumarate, propylene glycol alginate, octyl sulfosuccinate disodium, palmitoyl camitine.

11. The pharmaceutical composition of Sirolimus according to claim 8 wherein the composition further comprises pharmaceutically acceptable excipients selected from the group consisting of sugars, binders, diluents, lubricant/glidant, disintegrating agent, antioxidants and coloring agents.

12. The pharmaceutical composition of Sirolimus according to claim 8 wherein the composition is a capsule.

13. The pharmaceutical composition of Sirolimus according to claim 8 wherein the composition is a tablet.

14. The pharmaceutical composition of Sirolimus according to claim 13 wherein the tablet comprises an inert tablet core and a coating comprising Sirolimus.

15. The pharmaceutical composition of Sirolimus according to claim 14 wherein the tablet is prepared by a process comprising the step of:
a) Dissolving or dispersing a surface modifier in a solvent;
b) Dispersing Sirolimus in the dispersion/solution of step a);
c) Wet milling the dispersion to obtain desired particle size of Sirolimus;
d) Coating the inert tablet core with the Sirolimus dispersion of step c).

16. The pharmaceutical composition of Sirolimus according to claim 13 wherein the tablet is prepared by a process comprising the step of:
a) Dissolving or dispersing a surface modifier in a solvent;
b) Dispersing Sirolimus in dispersion/solution of step a);
c) Wet milling the dispersion to desired particle size;
d) Blending pharmaceutically acceptable inert excipients;
e) Granulating the excipients of step d) with dispersion of step c),
f) Drying and sizing the wet granules,
g) Compressing into a suitable size tablet.
